(19) Europäisches Patentamt
European Patent Office
Office européen des brevets

(11) Publication number: **0 168 738 B1**

(12) **EUROPEAN PATENT SPECIFICATION**

(45) Date of publication of patent specification: **16.09.92**

(51) Int. Cl.[5]: **C12Q 1/37**, C12Q 1/56

(21) Application number: **85108422.8**

(22) Date of filing: **08.07.85**

(54) **Single step protease inhibitor assay.**

(30) Priority: **20.07.84 US 632715**

(43) Date of publication of application:
**22.01.86 Bulletin 86/04**

(45) Publication of the grant of the patent:
**16.09.92 Bulletin 92/38**

(84) Designated Contracting States:
**DE FR GB**

(56) References cited:
**EP-A- 0 094 720**
**EP-A- 0 103 247**
**EP-A- 0 111 836**
**WO-A-84/02352**

**CLINICAL CHEMISTRY, vol. 28, no. 7, 1982, pages 1521-1524, Washington, US; G.F. KAPKE et al.: "Single-stage automated assay for Heparin"**

**CLINICAL CHEMISTRY, vol. 28, no. 11, November 1982, pages 2249-2253, Washington, US; W. PRELLWITZ et al.: "Two automated methods for plasma antithrombin III compared, and the clinical significance of the results"**

**Scand. j. Clin. Lab. Fnvest. Vol. 42, Suppl. 162, p. 42 (1982)**

**Symp. on Chromogenic Substrates at Wings's College Hospital Medical School, item 246 /1977)**

(73) Proprietor: **MILES INC.**
**1127 Myrtle Street**
**Elkhart Indiana 46514(US)**

(72) Inventor: **Schick, Lloyd A.**
**56392 Silvercrest Drive**
**Elkhart, IN 46514(US)**

(74) Representative: **Dänner, Klaus, Dr. et al**
**c/o Bayer AG Konzernverwaltung RP Patentabteilung**
**W-5090 Leverkusen 1 Bayerwerk(DE)**

EP 0 168 738 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

### FIELD OF THE INVENTION

The invention relates generally to a process for the preparation of unitary enzyme inhibitor assays. The assay so made is a single step assay for protease inhibitors which requires no preincubation of the inhibitor with its corresponding protease.

### UTILITY

Clinical interest in proteases and protease inhibitors is growing because of their diagnostic usefulness. Proteases are involved in such diverse physiological functions as coagulation, fibrinolysis, hormone production, fertilization and immuno-defense. Protease inhibitors serve to control the enzymes involved in these processes.

For example, antithrombin III is a primary inhibitor of the blood coagulation system and thus serves a vital role in maintaining hemostatic balance and control. Antithrombin III levels correlate well with an individual's predeposition to thrombosis. Therefore these levels provide the physician with a tool for assessing the prethrombotic state. Decreased concentrations of anti-thrombin III in plasma are associated with either an inherited hypercoagulable state, deep venous thrombosis or an oral contraceptive-induced alteration in hemostatic function. Lower concentrations are also seen in liver disease, in the nephrotic syndrome and with treatment with L-aspariginase.

### INFORMATION DISCLOSURE

Present assays for a protease inhibitor are based on the choice of a suitable peptide substrate (S) for its corresponding protease enzyme (E) so that the action of the protease on substrate in an aqueous environment leads to a detectable response. Upon the addition of the protease inhibitor (I) to the reaction system, this response is decreased.

$$I + (\text{excess})\ E \longrightarrow IE + E$$

$$H_2O + S \xrightarrow{\quad E \quad} \underset{\text{detectable response}}{P}$$

Previously it was believed that the reaction of the inhibitor with its corresponding protease must be allowed to go to completion prior to the addition of the substrate in order to achieve the desired relationship between inhibitor concentration and detectable response. For example, M.S. Scully et al. in *Clinica. Chimica Acta*, 79, (1977) 595-602 disclosed a method for the measurement of plasma antithrombin using the thrombin specific chromogenic peptide substrate, H-D-phe-pip-arg-*p*-nitroanilide. The antithrombin assay required the preincubation of the plasma sample with thrombin in buffer at 37 ° C. A portion of the preincubated solution was then added to a substrate-buffer mixture and that mixture was incubated again at 37 ° C before the addition of glacial acetic acid to stop the reaction. The amount of *p*-nitroaniline released was measured with a spectrophotometer. A kinetic study of the measurement of the heparin enhanced-antithrombin III/thrombin reaction rate in the presence of synthetic substrate was published by M.J. Griffith in *Thrombosis Research*, *25*: 245-253, 1982. This work was directed at the determination of the validity of kinetic models for the mechanism of reaction for heparin.

U.S. Patent No. 4,473,639, assigned commonly herein, disclosed a method for the determination of antithrombin III in mammalian blood plasma which comprises contacting the plasma with excess heparin and a three-layered reagent strip. In all of the published assays for protease inhibitor there is a time delay after the reaction of protease inhibitor and the protease which precedes the addition of a synthetic peptide substrate to assay residual protease. Surprisingly, it has now been found that no time delay to provide for

interaction of inhibitor and protease is necessary for the successfull determination of protease inhibitor. Documents EP-A-168 738, W0 84/2352 Kapke et al. (1982) Clin. Chem. 28 (7) 1521 - 1524, Scand. J. Clin. Lab. Invest Vol. 42, Suppl. 162, p 42 (1982) and Int. Symp. Chrom. Substr., item 246 are related to enzyme- and protease inhibitor tests but all of them are silent with regard to the present process.

## SUMMARY OF THE INVENTION

The invention provides a process for the preparation of an unitary single step assay of a protease inhibitor. The so made test does not require any preincubation step of the inhibitor with its corresponding protease. It is an unitary solid state test device useful for such an assay. The test device can be used for the single step assay of a protease inhibitor or for an activator (e.g. heparin) of a protease inhibitor.

The test device is prepared by incorporating a carrier matrix with a protease and its substrate in a manner which maintains the protease and substrate in substantially unreacted form. The test device incorporates the composition in a form which can be contacted with the aqueous test sample so that a detectable response is observed. The invention provides a single step solution assay method without any preincubation of protease with inhibitor or an unitary solid state device for a protease inhibitor or an ectivator of the inhibitor whereas current methods required the sequential addition of reagents with preincubation of the protease and inhibitor supposedly to assure a linear relationship between the observable response and the concentration of protease inhibitor.

## DETAILED DESCRIPTION OF THE DRAWINGS

### Figure 4

The reactivities of an unitary solid state test devices for the assay of antithrombin III are shown in Fig. 1. Test devices, prepared according to Example 1, were contacted with 1:3 dilutions of AT III standards containing 0, 25, 50, 70, 100 and 125 % normal human plasma. Reflectance measurements were taken every 5 seconds for 1 minute. The data was converted to K/S values and plotted vs. time, T, in seconds. The graph in Fig. 1 shows the effect of the addition of antithrombin III on the reactivity of the test device containing thrombin, S-2238 and heparin.

### Figure 2

The logarithm of the strip reactivity, K/S per time (T, in seconds) determined from graphs in Fig. 1 was plotted against time (T, in seconds) for various levels of antithrombin III (Concentrations of antithrombin III marked on the drawing in percent normal human plasma used as the test sample. Since AT III is a component of normal human plasma, such plasma was used to prepare standard test samples.) The data thus displayed is used to determine the half life, $t_{1/2}$, of the inhibition reaction and to calculate $k_{obs}$.

### Figure 3

The rate of thrombin inhibition by AT III as determined with a unitary test device comprising a carrier matrix incorporated with thrombin and S-2238 is shown in Fig. 3. $k_{obs}$ was plotted against the percent normal human plasma used as the test sample. The figure shows good linear correlation of the observable response, treated as described in the specification, and the concentration of antithrombin III in the sample.

## DETAILED DESCRIPTION OF THE INVENTION

Current unitary protease inhibitor assay methods require a time delay to allow the interaction of protease inhibitor with its corresponding protease prior to the addition of the protease substrate. This time delay is usually achieved by the sequential addition of reagents with preincubation. It has been shown that single step assay can be provided in an unitary solid state test device by incorporating a carrier matrix in such a way as to prevent premature interaction of the components of the test composition but to allow for essentially simultaneous contact of the protease and substrate with each other and with the test sample when wetted by the aqueous test sample. Although any aqueous test sample containing a protease inhibitor can be assayed the primary sample of interest is blood, in particular plasma.

### Test Components

A number of protease/protease inhibitor systems are known. For example antithrombin III inhibits all of the activated serum proteases of the coagulation system: Factors XIIa, XIa, IXa, Xa and thrombin. In addition protease/protease inhibitor systems of interest include elastase/$\alpha_1$-antitrypsin; $c_1$-esterase/$c_1$-esterase inhibitor; trypsin/inter-$\alpha$-trypsin inhibitor; plasmin/$\alpha_2$-antiplasmin and chymotrypsin/$\alpha_1$-anti-chymotrypsin.

In the case of the thrombin/antithrombin III system, heparin, an activator of the inhibitor, is advantageously added to the reaction to increase the binding of antithrombin III to thrombin. When the thrombin/antithrombin III/heparin system is used either heparin or antithrombin III can be determined quantitatively depending on the components of the assay composition used.

The substrate should be chosen with two criteria in mind; first the substrate should be as specific for the protease used in the assay as possible; second the substrate will determine the character of the detectable response. The action of the protease on the substrate cleaves the substrate. The cleavage product can itself provide a colorimetric or fluorimetric response, or it can react with an coupling component to provide a detectable response.

Many synthetic substrates have been developed for various proteases for which a cleavage product is colored or fluorescent. Table I below provides an overview of protease/protease inhibitor systems with some commercially available substrates. Substrates with an "S" prefix are manufactured by Kabi Diagnostica, Stockholm, Sweden. Substrates with a suffix "MCA" are flurogenic methyl-coumarin amide esters available from Peninsula Laboratories, Belmont, California. The suffix "pNA" indicates the *p*-nitroanilide derivative which when cleaved produces the yellow *p*-nitroaniline.

TABLE I

| Protease inhibitor | Protease | Substrate |
|---|---|---|
| Antithrombin III | Factor Xa | N-Bz-Ile-glu-gly-arg-pNA (S-2222)<br>BOC-Ile-glu-gly-arg-MCA |
| | thrombin | D-phe-pip-arg-pNA (S-2238)<br>Boc-val-pro-arg-MCA |
| $\alpha_1$-antitrypsin | elastase | L-Pyroglutanyl-pro-val-pNA (S-2484)<br>suc-ala-pro-ala-MCA |
| $c_1$-esterase inhibitor | $c_1$-esterase | suc(ala)$_3$-pNA |
| inter-$\alpha$-trypsin inhibitor | trypsin | H-D-Ile-pro-arg-pNA (S-2288)<br>Boc-phe-ser-arg-MCA<br>Bz-arg-pNA |
| $\alpha_1$-antichymotrypsin | chymotrypsin | 3-carbomethoxy-propionyl-arg-pro-tyr-pNA [S-2586)<br>Suc-ala-ala-pro-phe-MCA |
| $\alpha_2$-antiplasmin | plasmin | H-D-val-leu-lys-pNA (S-2251)<br>Boc-val-leu-lys-MCA |

Other substrates do not produce a detectable response directly on cleavage, but can be detected when the cleavage product reacts with an additionally provided coupling component. Thiol esters have been described as substrates for proteolytic enzymes (e.g. see Green *et al Anal. Biochem. 93* 223 (1979). For example the ester, N-$_\alpha$-benzyloxy carbonyl-L-lysine thiobenzyl ester hydrochloride, can be used as a substrate for thrombin, plasmin or trypsin. When a coupling component such as 5,5'-dithiobis(2-nitro) benzoic acid (Ellman's reagent) is added to the test composition,there is a detectable chromogenic response. Other coupling components can be used in combination with a thiobenzyl ester substrate. These include 2,2'-dithiopyridine and 4,4'-dithiopyridine. In addition, tripeptide thiol ester substrates can be used for the quantitative determination of proteolytic enzymes as described in U.S. Patent No. 4,434,096. The free thiol group released during enzymic reaction is measured photometrically after reaction with a coupling component as described above.

The result of the present invention is a test device which is composed of a carrier matrix incorporated with a protease and its substrate in such a way as to prevent interaction of the protease and its substrate prior to contact with the aqueous test sample.

The carrier matrix can be any substance capable of being incorporated with the components of the test

composition, as long as it is substantially inert with respect to the test composition, porous and/or absorbent relative to the aqueous sample to be tested. The expression "carrier matrix" refers to either bibulous or nonbibulous matrices which are insoluble in and maintain their structural integrity when exposed to water or to other physiological fluids. Suitable bibulous matrices which can be used include paper, cellulose, wood, synthetic resin fleeces, woven and nonwoven fabrics and the like. Nonbibulous matrices includes glass fiber, polymer films, preformed or microporous membranes and organoplastic materials such as polypropylene and the like.

Other matrix formats are contemplated, including the use of a commercially available preformed porous membranes or microporous membranes formed by techniques such as phase inversion. Polymer film matrices such as films produced by latex formulations based on latex polymer suspensions, for example the 60:40 copolymer of styrene and butadiene, or other natural or synthetic polymers or mixtures thereof. Examples of such film formulations can be found in U.S. Patent Nos. 3,630,957 and 4,312,834.

The unitary solid state test strip or test device is prepared by incorporation of a carrier matrix with drying between incorporation steps. When a whole blood sample is tested, the impregnated carrier matrix can be coated to allow excess sample to be washed or wiped off.

Incorporation can be accomplished by any method such as dipping, spreading or spraying which allows the carrier matrix to be incorporated with the protease and the substrate in a manner which will prevent premature interaction but which will allow essentially simultaneous contact with the protease substrate and the test sample when wetted by the aqueous sample. This can be accomplished by impregnating a paper carrier matrix with an aqueous solution containing the protease, drying; and then impregnating the dried carrier with a nonaqueous solution containing the substrate, and drying. Optionally components such as an activator or coupling component can be incorporated with whichever solution is most feasible as determined by their solubility in aqueous or nonaqueous solvents.

Drying can be accomplished by any means which will not deleteriously affect the incorporated composition, usually by means of an air oven. The dried paper can thereafter be cut and mounted on one end of a support member, for example, a rigid or semi-rigid polystyrene film strip. Mounting of the paper on the strip can be accomplished through use of a double-faced adhesive tape, such as that commercially available from the 3M Co. St. Paul, MN. as DOUBLE STICK®. The support member provides a convenient handle which facilitates use of the test. The test device format is a particularly convenient form with which to practice the assay method of the present invention. Reactants can be incorporated into a single carrier matrix such as paper and yet maintained without prior reaction. The test device is simply contacted with a sample and a detectable result recorded. Results and data manipulation to determine the concentration of protease inhibitor from the detectable result and are described infra at Results.

3. Concentration Ranges of Test Components

The concentration ranges of test components in the assay depend on the clinical range of interest for the particular protease inhibitor. Table II indicates the normal range for protease inhibitors in human plasma. Values outside this range are considered abnormal and therefore of clinical interest.

TABLE II

| Protease Inhibitor | Normal Range (mg/100 mL) |
|---|---|
| $\alpha_1$ -antitrypsin | 200-400 |
| $\alpha_1$-antichymotrypsin | 30-60 |
| inter-$\alpha$-trypsin inhibitor | 20-70 |
| antithrombin III | 17-30 |
| $c_1$-Inactivator | 15-35 |
| $\alpha_2$-macroglobulin | 150-350 |
| $\alpha_2$-antiplasmin | 9-12 |

The following provides a guide to the working and preferred concentration ranges for components in the reagent solution used to prepare the test device for the determination of antithrombin III.

| | working | preferred |
|---|---|---|
| protease (thrombin) | 0.2-50 NIH units/mL | 5-20 NIH units/mL |
| substrate | 0.2-5 mM | 0.5-2 mM |
| protease inhibitor (antithrombin III 1:3 dilution of plasma) | 0-150% NHP | 20-150% NHP |
| activator (heparin) | 0.2-100 USP units/mL | 0.5-30 USP units/mL |

In a particularly preferred embodiment a unitary solid state test device for antithrombin III is prepared by sequentially incorporating a carrier matrix with an aqueous solution containing from 5 to 10 NIH units/mL thrombin and 1 to 10 USP units/ml heparin and drying and incorporating the dried matrix with a nonaqueous solution containing from 1 to 2 mM chromogenic thrombin substrate and drying. Reagent concentrations required for the determination of any other protease inhibitor once again depend primarily on the protease inhibitor/protease pair of interest and on the sensitivity of the detection method chosen. A similar procedure as that outlined for determining solution reagent concentrations would be used to determine preferred concentrations to prepare a test device. However, it is expected that such concentrations would fall within the working concentrations given for antithrombin III.

The same method can be used to assay and to prepare devices for the determination of heparin if antithrombin III is incorporated into the test composition or test device and heparin is deleted. For this reason, working and preferred concentration ranges for antithrombin III are given above even though it is contemplated that antithrombin III, or another protease inhibitor, will be the analyte of interest and as such will not be incorporated into the test device or combined into the single assay reagent solution.

The test device is advantageously used by simply contacting the sample and the test device, whereby a detectable response results. Contact with the test device can be made by dipping, pipette or swab.

Results

When the assay composition is incorporated into a carrier matrix to prepare a unitary solid state test device, reflectance is used to measure the detectable chromogenic response. Reflectance was monitored every 5 seconds for 2 minutes using an Ames SERALYZER® spectrophotometer with a 405 nm interference filter. When the SERALYZER is connected to a Hewlett Packard HP 85 computer, graphical displays of the data can be obtained directly.

The reflectance measurements were evaluated with a simplified form of the well known Kubelka-Munk equation (see Gustav Kortum, "Reflectance Spectroscopy", pp 106-111, Springer Verlag, NY (1969)):

$$K/S = \frac{(1-R)^2}{2R}$$

in which R is the fraction of reflectance from the test device, K is a constant and S is the light scattering coefficient of the particular reflecting medium. K/S is related to the concentration of the absorbing species, usually the cleavage product formed by the interaction of the protease with the substrate. K/S was plotted against time in seconds, analogous to the plot of absorbance against time for the liquid assay. Semilogarithmic plots of the reactivity (change in K/S per second determined from the K/S vs. time plots) against time in seconds were used to determine the half life ($t_{\frac{1}{2}}$) of the inhibition reaction. As in the liquid assay, the observed rate constant ($k_{obs}$) was calculated from the relationship

$k_{obs} = 0.693/t_{\frac{1}{2}}$

A linear relationship of protease inhibitor concentration in the sample solution with the observable response was established. Unknown samples can be determined with similar calculations and comparison to a standard curve.

The following examples describe experiments which were performed in developing the present invention. While the examples serve to illustrate the invention, they are not to be interpreted as limiting its scope which is defined solely by the claims appended hereto. One skilled in the art will be able to make such variations, substitutions and changes in the components of the composition and ingredients and reaction parameters as may seem desirable.

EXAMPLES

The following abbreviations are used in the specification for convenience.

| | |
|---|---|
| dL | deciliter |
| mL | milliliter |
| μL | microliter |
| M | molar |
| mM | millimolar |
| nM | nanomolar |
| μM | micromolar |
| mg | milligram |
| gm | gram |
| nm | nanometers |
| °C | degrees centigrade |
| IU | International Units - the amount of enzyme that will catalyze the hydrolysis of one micromole of substrate per minute under the conditions of the assay |
| NHP | Normal Human Plasma |
| NIH Unit | the amount of thrombin that will clot a 250 mg% fibrinogen solution in 15 seconds at 37°C |
| U.S.P. Heparin Unit | Unit of potency for heparin defined in relationship to a USP reference standard |
| AT III | Antithrombin III |
| $t_{\frac{1}{2}}$ | half life of the reactant |
| $k_{obs}$ | observed rate constant |
| v | reaction velocity |
| S-2238 | D-phe-pip-arg-pNA, a synthetic chromogenic substrate for thrombin, obtained from Kabi Diagnostica, Stockholm Sweden |
| thrombin | bovine thrombin obtained from Miles Scientific, Naperville, Ill. |
| heparin | porcine intestinal mucosal heparin obtained from Sigma Chemical Co., St. Louis MO. |
| EDTA | Ethylenediaminetetraacetic acid |
| Tween 80 | polyoxyethylene(20) sorbitan mono-oleate obtained from Sigma Chemical Co., St. Louis MO. |
| Gantrez ES-225 | ethylester of methylvinylether/maleic anhydride copolymer, obtained from GAF Corp.,New York, N.Y. |
| DMF | Dimethylformamide |
| Tris | *tris*(hydroxymethyl)aminomethane |
| Emulphor® ON-870 | polyoxyethylated oelyl alcohol, GAF Corp., New York, N.Y. |

Substrate nomenclature

| | |
|---|---|
| BOC | benzyloxycarbonyl |
| p-NA | para-nitroanilide |
| Bz | Benzyl |
| suc | succinyl |
| glu | glutamic acid |
| Ile | isoleucine |
| gly | glycine |
| arg | arginine |
| pro | proline |
| val | valine |
| phe | phenylalanine |
| ser | serine |
| lys | lysine |
| pip | pipecolic acid |

Percentages used indicate weight in grams per 100 milliliters of the solution (% w/v) unless otherwise indicated.

Example 1: Unitary Test Device Useful For Antithrombin III Assay

A solid state test device incorporating all components necessary to perform a single-step assay for antithrombin III was prepared as follows.

Whatman 54 filter paper was dipped in an aqueous solution of 0.2 M Tris, 0.175 M sodium chloride and 7.5 mM EDTA (pH 8.4) containing 1% bovine serum albumin, 10 NIH units/mL thrombin and 30 units/mL heparin. The impregnated paper was dried at 50 °C for 15 minutes and then dipped in a nonaqueous solution of one part dimethylformamide to four parts ethanol containing 2 mM S-2238, 0.1% Tween 80 and 1% Gantrez ES-225.

Standards for antithrombin III were prepared by diluting normal human plasma (NHP) to produce solutions containing 0, 10, 20, 40, 60, 80 and 100 % NHP. The mean concentration of AT III in NHP is 23.5 mg/1oo ml. AT III concentrations correspond to the % NHP values as follows:

| Standard % NHP | AT III Concentration (mg/100 mL) |
|---|---|
| 0 | 0 |
| 10 | 2.35 |
| 20 | 4.70 |
| 40 | 9.40 |
| 60 | 14.10 |
| 80 | 18.80 |
| 100 | 23.5 |

All test samples and standards were diluted one part to two parts of normal saline prior to assay due to the measurement range of the available instrumentation.

A 30 µL aliquot of diluted plasma was pipetted onto the test device and the reflectance monitored every 5 seconds for 2 minutes using a Ames SERALYZER(R) spectrophotometer with a 405 nm interference filter. (Fig. 1 ) Semilogarthmic plots were used to determine half time ($t_{\frac{1}{2}}$) for the inhibition reaction. The observed rate constant ($k_{obs}$) was calculated from the relationship $k_{obs} = 0.693/t_{\frac{1}{2}}$. The plot of $k_{obs}$ against nanomolar antithrombin III concentration (Fig. 3) indicates that the rate of thrombin inhibition is indeed a linear function of antithrombin III concentration. Semi-log plots of reactivity (delta K/S per second) with time were used to determine t $\frac{1}{2}$ the half-life of thrombin activity (see Fig. 2). The observed rate constant $k_{obs}$, was calculated and plotted versus the antithrombin III concentration in the standard to prepare a response curve. (see Fig. 3) Levels of antithrombin III in test plasmas were determined from the response curve.

## Claims

1. A process for the preparation of a unitary test device for the single step assay of a protease inhibitor in an aqueous test sample without preincubation of the inhibitor with its corresponding protease which process comprises the steps of:
   a) impregnating a carrier matrix with an aqueous solution containing the protease;
   b) drying the incorporated matrix;
   c) incorporating the dried matrix with a nonaqueous solution containing the substrate; and
   d) drying.

2. The process of claim 1 in which the protease is thrombin and the substrate is a synthetic chromogenic substrate for thrombin.

3. The process of claim 2 wherein the test device is prepared using an aqueous solution containing from about 0.2 to abut 50 NIH units/mL thrombin and optionally from about 0.2 to about 100 USP units/mL heparin, and with a nonaqueous solution containing from about 0.2 to about 5 M of a synthetic chromogenic substrate for thrombin.

4. Use of the test device prepared according to any of the process claims 1-3 for the determination of a protease inhibitor.

## Patentansprüche

1. Verfahren zur Herstellung einer einheitlichen Testvorrichtung für den einstufigen Test eines Proteaseinhibitors in einer wässrigen Testprobe ohne Vorinkubation des Inhibitors mit dessen entsprechender

Protease, umfassend die Schritte:

(a) Imprägnieren einer Trägermatrix mit einer wässrigen, proteasehaltigen Lösung;
(b) Trocknen der inkorporierten Matrix;
(c) Inkorporieren einer nicht-wässrigen, das Substrat enthaltenden Lösung in die getrocknete Matrix; und
(d) Trocknen.

**2.** Verfahren nach Anspruch 1, bei dem die Protease Thrombin ist und das Substrat ein synthetisches, chromogenes Substrat für Thrombin ist.

**3.** Verfahren nach Anspruch 2, bei dem die Testvorrichtung unter Verwendung einer wässrigen Lösung, die etwa 0,2 bis etwa 50 NIH-Einheiten/ml Thrombin und gewünschtenfalls etwa 0,2 bis etwa 100 USP-Einheiten/ml Heparin enthält, und mit einer nicht-wässrigen Lösung, die etwa 0,2 bis etwa 5 M eines synthetischen, chromogenen Substrates für Thrombin enthält, hergestellt wird.

**4.** Verwendung der nach einem der Verfahrensansprüche 1 bis 3 hergestellten Testvorrichtung für die Bestimmung eines Proteaseinhibitors.

**Revendications**

**1.** Procédé de préparation d'un dispositif analytique unitaire pour le dosage en une seule étape d'un inhibiteur de protéase dans un échantillon analytique aqueux, sans pré-incubation de l'inhibiteur avec sa protéase correspondante, procédé qui comprend les étapes consistant :

a) à imprégner une matrice de support avec une solution aqueuse contenant la protéase ;
b) à sécher la matrice ayant été soumise à cette incorporation ;
c) à incorporer à la matrice séchée une solution non aqueuse contenant le substrat ; et
d) à effectuer un séchage.

**2.** Procédé suivant la revendication 1, dans lequel la protéase est la thrombine et le substrat est un substrat chromogène synthétique pour la thrombine.

**3.** Procédé suivant la revendication 2, dans lequel le dispositif analytique est préparé au moyen d'une solution aqueuse contenant environ 0,2 à environ 50 unités NIH/ml de thrombine et, facultativement, environ 0,2 à environ 100 unités USP/ml d'héparine, et au moyen d'une solution non aqueuse contenant environ 0,2 à 5 M d'un substrat chromogène synthétique pour la thrombine.

**4.** Utilisation du dispositif analytique préparé suivant l'une quelconque des revendications de procédé 1 à 3 pour le dosage d'un inhibiteur de protéase.

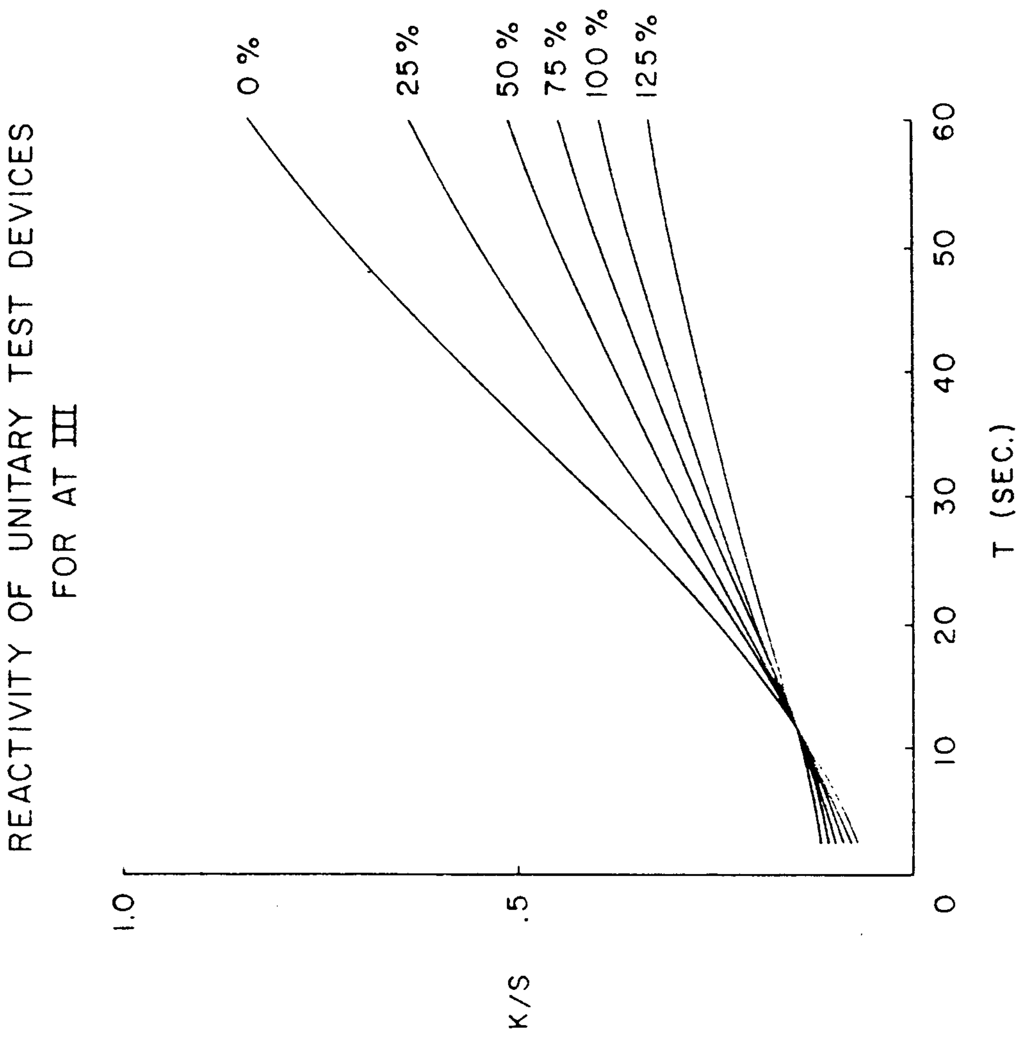
REACTIVITY OF UNITARY TEST DEVICES
FOR AT III
K/S
1.0
.5
0
10
20
30
40
50
60
T (SEC.)
0 %
25 %
50 %
75 %
100 %
125 %

FIG. 1

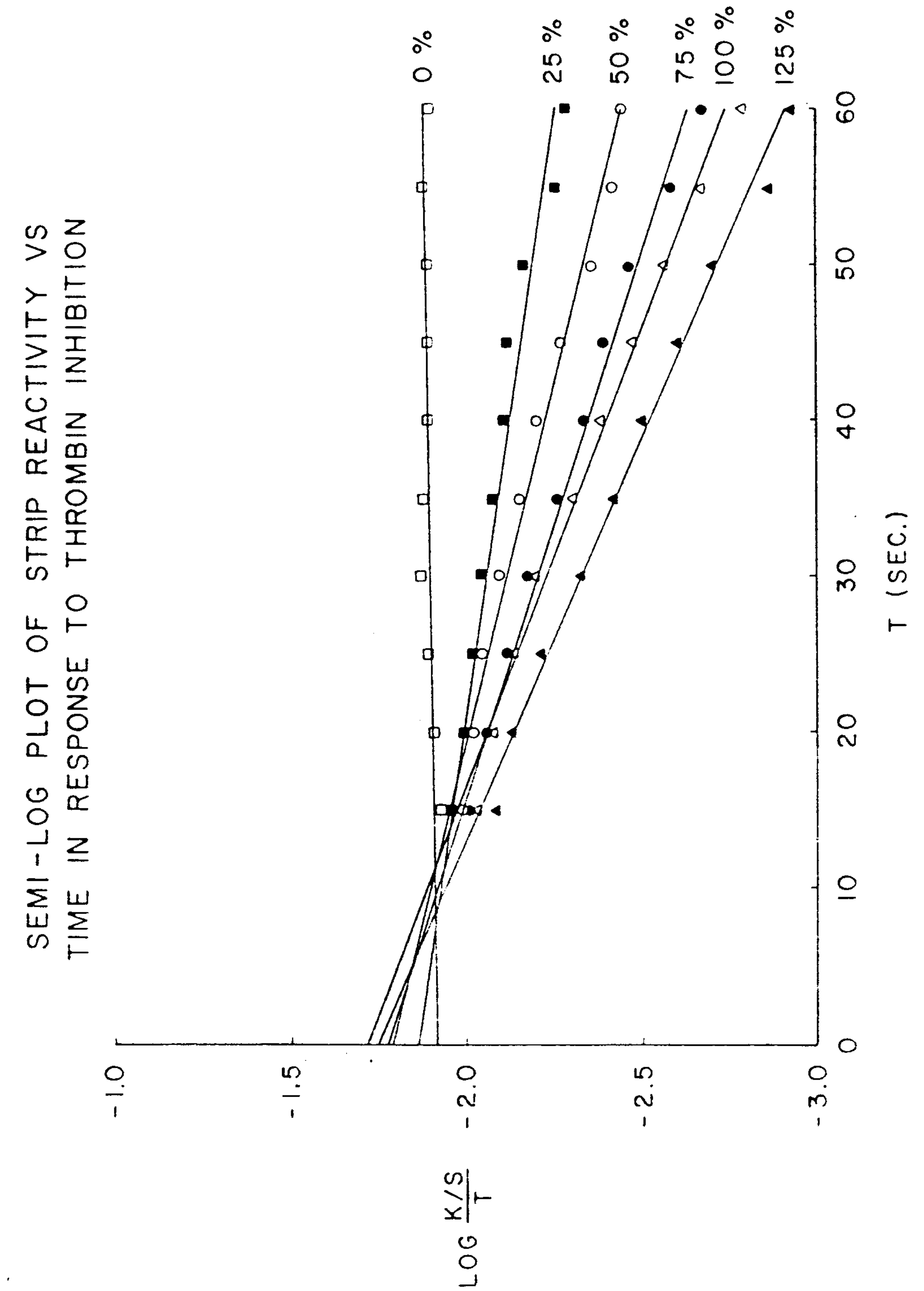
SEMI-LOG PLOT OF STRIP REACTIVITY VS
TIME IN RESPONSE TO THROMBIN INHIBITION
LOG K/S / T
-1.0
-1.5
-2.0
-2.5
-3.0
0
10
20
30
40
50
60
T (SEC.)
0 %
25 %
50 %
75 %
100 %
125 %

FIG. 2

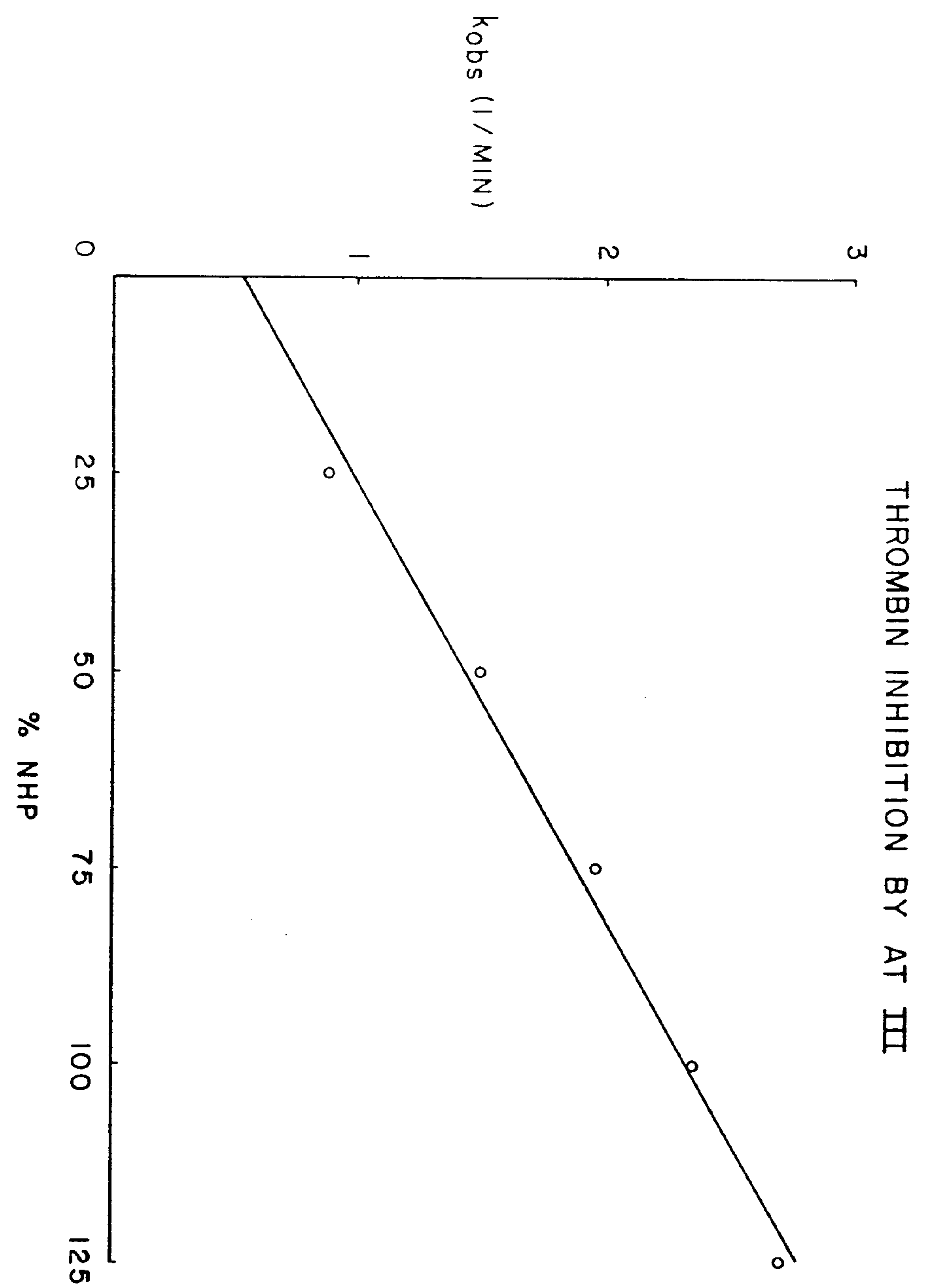
UNITARY TEST DEVICE: RATE OF
THROMBIN INHIBITION BY AT III
kobs (1/MIN)
0
1
2
3
25
50
75
100
125
% NHP

FIG. 3